# EUROPEAN PATENT APPLICATION

(11) **EP 1 178 113 A1**
(43) Date of publication of application: **06.02.2002**
(21) Application number: 00116936.6
(22) Date of filing: 04.08.2000
(51) Int. Cl.: C12N 15/53, C12N 15/82, C12P 13/24, C12N 5/10, C12Q 1/26, A01H 5/00

(54) **Plant protein with pyrroline-5-carboxylat-dehydrogenase activity**

(71) Applicant: Sympore GmbH, 72076 Tübingen (DE)
(72) Inventor: Funck, Dietmar, 72076 Tübingen (DE); Deuschle, Karen, 72760 Reutlingen (DE); Hellmann, Hanjo, 33617 Bielefeld (DE); Frommer, Wolf Bernd, 72076 Tübingen (DE)
(74) Representative: Patentanwälte Ruff, Wilhelm, Beier, Dauster & Partner

(57) **Abstract**

The present invention relates to nucleic acid sequences encoding an amino acid sequence with pyrroline-5-carboxylate dehydrogenase activity naturally occuring in plants. Furthermore, the present invention provides methods for the detection of pyrroline-5-carboxylate dehydrogenase activity and for the production of transgenic plants with modified pyrroline-5-carboxylate dehydrogenase activity. Therefore, the present invention is useful for the development of an active substance, especially a fungicide and/or herbicide, in plants.

## Description

The present invention relates generally to genetic sequences which encode an amino acid sequence with a pyrroline-5-carboxylate dehydrogenase activity naturally occurring in plants. In particular, the present invention relates to nucleic acid molecules, peptides which are encoded by said nucleic acid molecules and which are involved in proline degradation, vectors comprising these nucleic acid molecules and host cells comprising such vectors or nucleic acid molecules. The present invention further provides methods for the detection of pyrroline-5-carboxylate dehydrogenase activity, especially in plants and for the production of transgenic plants with modified pyrroline-5-carboxylate dehydrogenase activity. The present invention is particularly useful for the development of an active substance, especially a fungicide and/or herbicide, in plants.

As is known, proline (PRO, P) is a non-essential amino acid which is synthesized from glutamic acid (Glu) via pyrroline-5-carboxylate (P5C). This reaction, e.g. in plants is catalysed by two enzymes, P5C synthetase (P5CS) and P5C reductase (P5CR). Proline is metabolized to glutamic acid (Glu) via pyrroline-5-carboxylate (P5C) by two enzymes, proline dehydrogenase (ProDH) and pyrroline-5-carboxylate dehydrogenase (P5CDH).

In plants several mechanisms are used for survival in adverse environments such as drought, high and low temperature and salinity. In many plants, osmotic stress induces a rapid accumulation of proline mainly by de novo synthesis from glutamic acid. For instant, in Arabidopsis thaliana proline can account for up to 20 % of the free amino acid pool after salt stress. This accumulation of proline in stressed plants is caused both by activation of the biosynthesis of proline and by inactivation of the degradation of proline. The catabolism of proline is inhibited when proline accumulates during dehydration and it is activated when rehydration occurs. Under dehydration conditions, when expression of the gene for P5CS is strongly induced, expression of the gene for ProDH is inhibited by contrast, under rehydration conditions, when the expression of the gene for ProDH is strongly induced, the expression of the gene for P5CS is inhibited. Furthermore, the gene for the P5CS is induced by high sugar concentrations but repressed by proline, and proline dehydrogenase (ProDH) is inversely regulated. The amino acid proline is also discussed as a protectant of macromolecules or even as a scavenger of hydroxyl radicals (1-3). Furthermore, proline can serve as a rapidly available source of nitrogen, carbon, and reduction equivalents during recovery from stress (4, 5). In principle the accumulation of proline can be achieved by de novo synthesis in the affected cells (6, 7), by decreased degradation, or by specific transport systems that distribute proline to the locations of need (8, 9).

Proline is synthesized in the cytosol (10-12), and P5CS is the rate limiting enzyme in this reaction. The expression of P5CS is feedback inhibited by proline, but induced under conditions of stress (13). Degradation of proline to pyrroline-5-carboxylate takes places in mitochondria (14). Expression of ProDH is induced by proline and hypoosmotic conditions, but repressed by water deficiency and is thought to be the rate limiting step of degradation (14). De novo synthesis, catabolism, and transport of proline are highly regulated by both abiotic stress and cellular proline concentrations. Often the accumulation of proline under osmotic stress is accompanied by an increase of the soluble sugar content (15-19). It was shown by Hellmann et al. (20), that even moderate exogenous proline application in the absence of stress are toxic for Arabidopsis. In this publication it was shown, that probably not proline by its own, but the intermediate P5C caused the toxicity. The exogenous proline application resulted in the increased ProDH expression and therefore likely in a high turnover of imported proline and the accumulation of the toxic intermediate. The key enzyme for the degradation of pyrroline-5-carboxylate and therefore for the toxicity caused by exogenous proline application is the enzyme pyrroline-5-carboxylate dehydrogenase which catalyses the second step of the proline degradation, the conversion of pyrroline-5-carboxylate to glutamic acid.

It is known from bacteria, that both enzymatic functions in proline degradation, the metabolization of proline to pyrroline-5-carboxylate and the degradation of these intermediates to glutamic acid, are linked to one polypeptide with two catalytic domains. In yeast, both genes are characterized which are involved in the conversion of proline to glutamic acid, including Put2, the yeast gene for P5CDH. Nevertheless, in plants like Arabidopsis and several other plants only the gene for ProDH (proline dehydrogenase) is isolated (14, 10, 21), a plant gene for P5CDH was not identified previously.

Thus, the object of the invention is to identify and characterize the enzyme pyrroline-5-carboxylate dehydrogenase and therefore to make available this enzyme for a number of applications, especially on the field of agriculture. This object and other objects are solved by the molecules according to claim 1, and the subject-matter of independent claims 8, 9, 10, 11, 12, 13, 15, and 16. Preferred embodiments are given in the dependent claims 2 to 7, 14, and 17. The content of all these claims is hereby incorporated to the description by reference.

Accordingly, one aspect of the present invention comprises an nucleic acid molecule encoding an amino acid sequence naturally occuring in plants with a pyrroline-5-carboxylate dehydrogenase activity.

A preferred embodiment of the present invention, provides a nucleic acid molecule, which is at least a part of a sequence according to SEQ ID NO. 1 or a complementary strand thereof.

For the purpose of clarification, fragments or complementary strands thereof includes also antisense molecules. These molecules are nucleic acid molecules which are capable of forming a double-stranded duplex with a mRNA molecule, thereby preventing its translation into a polypeptide.

Preferably the percentage similarity to the sequence set forth in SEQ ID NO. 1 is at least 60 %. More preferably, the percentage similarity is at least 80 %. Still more preferably, the percentage similarity is at least 90 % to at least a part of a sequence or to a complementary strand thereof. Those skilled in the art are aware that the percentage nucleotid sequence identity between homologous genomic genes isolated from different species is highest within the region comprising the structural gene, in particular the coding region thereof. The regions of highest identity are usually limited to short parts of approximately 6 to 10 nucleotides in length.

For the purposes of nomenclature, the nucleic acid molecule set forth in SEQ ID NO. 1 is the P5CDH cDNA sequence of Arabidopsis thaliana.

In an alternative preferred embodiment, the present invention provides an nucleic acid molecule, wherein said nucleic acid molecule hybridizes under at least low stringency conditions to the nucleotide sequence set forth in SEQ ID NO. 1 or a complementary strand thereof.

For the purposes of defining the level of stringency, a low stringency is defined herein as being hybridisation and/or a wash carried out in 6 x SSC buffer 0.1 % (w/v) SDS at 28 °C. Generally, the stringency is increased by reducing the concentration of SSC buffer, and/or increasing the concentration of SDS and/or increasing the temperature of the hybridisation and/or wash. Conditions for hybridisations and washes are well understood by one normally skilled in the art. For the purposes of clarification, (to parameters affecting hybridisation between nucleic acid molecules), reference is found in (22), which is herein incorporated by reference.

In a particularly preferred embodiment of the invention, a nucleic acid molecule that encodes at least a part of an amino acid sequence according to SEQ ID NO. 2 is provided.

For the purposes of nomenclature the sequence shown in SEQ ID NO. 2 relates to the amino acid sequence of pyrroline-5-carboxylate dehydrogenase of Arabidopsis thaliana which is encoded by the SEQ ID NO. 1.

In another preferred embodiment of the invention, the nucleic acid molecule is a DNA molecule, especially a cDNA or genomic DNA molecule or even a RNA molecule.

In a particularly preferred embodiment of the present invention, the nucleic acid molecule is combined with a regulatary element like binding sites for transcription factors, silencers, enhancers or promoters, preferably by a promoter suited for organ specific expression in organisms, especially in plants. There is a broad range of promoters like the lacZ (β-galoctosidase), cat (chloramphenicol-acetyltransferase), dhfr (dihydrofolatreductase), lux (luciferase) or the uidA (β-glucuronidase) promoter known by those who are skilled in the art.

In another aspect according to the present invention, vectors comprising a nucleic acid molecule as described above or fragments thereof are provided. There is a huge number of specific vectors. They are known by those who are skilled in the art and they are commercially available by biotechnology companies like Stratagene, Invitrogen, Qiagen etc.

In another aspect according to the present invention, host cells comprising at least one nucleic acid molecule or a vector comprising said nucleic acid molecule are claimed. These cells are normally transfected by standard molecular biology techniques like electroporation, lipofectamin transfection etc.. The technique used for a given plant species or specific type of plant tissue depends on the known successful techniques. Means for introducing recombinant DNA into plant tissue include, but are not limited to, transformation, electroporation or microinjection of the DNA or T-DNA mediated transfer from Agrobacterium to the plant tissue. Representative T-DNA vector systems are known by those skilled in the art. Once introduced into the plant tissue, the expression of the nucleic acid may be assayed in a transient expression systems, or it may be determined after selection for stable integration within the plant genome. Techniques are known for the in vitro culture of plant tissue, and for regeneration into whole plants. Procedures for transferring the introduced nucleic acid from the originally transformed plant into commercially useful cultivars are known to those skilled in the art.

In another aspect a method is provided to screen plants or parts thereof with respect to pyrroline-5-carboxylate dehydrogenase activity. The method for screening plants comprises a) cultivating plants or parts thereof on proline and/or pyrroline-5-carboxylate containing substrate, and b) monitoring sensitivity against proline or pyrroline-5-carboxylate by monitoring the growth on said substrate. Conditions for cultivation (20) and screening of plants or parts thereof, e.g. by physiological analyses or optical selection of plants showing more/less viability on substrate containing medium, are known by those who are skilled in the art.

In a most preferred aspect according to the invention it is provided a method to develop an active substance, especially a fungicide and/or herbicide, wherein said method is characterized by:
a) transforming cells, especially yeast cells deficient in endogenous pyrroline-5-carboxylate dehydrogenase activity, with a nucleic acid molecule encoding an amino acid sequence naturally occuring in plants with pyrroline-5-carboxylate dehydrogenase activity,
b) incubating the cells with a substance potentially altering the activity of the pyrroline-5-carboxylate dehydrogenase transformed into the cells, and
c) monitoring the activity of the pyrroline-5-carboxylate dehydrogenase to identify active substances.

According to this method, the investigation according to step c) is preferably performed by monitoring the growth on proline- and/or pyrroline-5-carboxylate containing substrate. Also the use of a nucleic acid molecule, a vector containing a nucleic acid molecule, or an amino acid sequence as targets for the development of an active substance, especially a fungicide and/or herbicide, is possible. In mammals accumulation of P5C is related to processes leading to apoptosis (23). Studies of proline toxicity in Arabidopsis suggest a similar effect in plants (20). Inhibitors of plant P5CDH expression or activity can therefore serve as highly efficient herbicides. A growth assay with yeast cells depending on a plant P5CDH for the utilization of proline as the sole source of nitrogen can be used for high throughput screening of large chemical libraries to identify potential P5CDH inhibitors. With yeast and human P5CDHs or even P5CDHs from different plant species as controls, specific herbicides can be identified that do not harm any other organisms unintendedly. It was also shown that treatment of seedlings with proline in hypoosmotic conditions is toxic even for wild type plants. The overexpression of P5CDH will overcome proline toxicity and is therefore providing a selectable marker system without the use of genes from other organisms.

The invention finally comprises the use of an inventive nucleic acid molecule, an inventive vector comprising said nucleic acid molecule, or the amino acid sequence encoded by said nucleic acid molecule for the investigation of proline pathways, especially the degradation of proline, especially in plants. This investigation may be characterised in that direct or indirect modulators, especially inhibitors, of pyrroline-5-carboxylate dehydrogenase are to be identified.

The described features and further features of the invention can be gathered from the following description of preferred embodiment in conjunction with the sub-claims. The individual features can be implemented separately or in the form of subcombinations.

### MATERIALS AND METHODS

In the drawing it is shown:

Figure 1: Yeast expression vector pFL6I with inserted cDNA of AtPut2.

Figure 2: Constructs used for overexpression of yeast Put2 in Arabidopsis thaliana. Only the parts of the plasmets between the T-DNA borders are shown.

### Material and Methods:

All chemicals, unless stated otherwise, were analytical grade and purchased from Sigma (St. Louis, MO, USA). Media for bacteria, yeast, and plant culture were purchased from Becton Dickinson (Sparks, MD, USA) if not stated otherwise.

### Bacteria

E. coli cells (strain XL1-Blue (Stratagene, La Jolla, CA, USA) and transformants thereof) were grown at 37°C in LB medium solidified with 15 g/l Bacto agar if appropriate (24). Agrobacterium tumefaciens cells (strain pGV2260 (25) and transformants thereof) were grown in YEB Medium (26) at 28°C.

### Transformation using electroporation

40 µl of a suspension of electrocompetent cells (24) were mixed with 1 - 10 ng plasmid DNA. Electroporation was carried out using a BTX TransPorator Plus (BTX Inc., San Diego, Ca, USA) following the manufacturers instructions. The bacterial membrane was porated by applying an electric field of approximately 13.5 or 15 V/cm (for E. coli or A. tumefaciens cells, respectively) for 5 - 6 msec. Directly afterwards, cells were incubated under rotation for 30 min at 37°C in LB medium without antibiotics and then plated on medium supplemented with the appropriate antibiotics.

### Plasmid preparation

Plasmids were isolated by the alkaline lysis method (24) and stored in TE buffer.

### Yeast

Saccharomyces cerevisiae cells were grown in either full (10 g/l Bacto yeast extract, 20 g/l Bacto peptone, 20 g/l glucose) or synthetic minimal medium (1.7 g/l yeast nitrogen base without amino acids, 20 g/l glucose, 0.1% (w/v) of either urea or proline) solidified with 20 g/l agar (bacteriological agar Nr. 1, OXOID Ltd., Basingstoke, GB) where appropriate.

The following strains were used:
23344c (Mata, ura 3), wildtype strain except for ura3 mutation
MB1472 Genotype: MATa, ura3-52, trp1, put2::TRP1 (The trp1 gene replaces codons 15-538 of the Put2 gene). Equivalent strains can be used or created by standard methods (e.g. (27))

### Yeast transformation:

Yeast cells were transformed using the lithium acetate/single stranded carrier DNA/polyethylene glycol method (28).

### Plasmid isolation:

A 1.5 ml overnight culture was pelleted by centrifugation in a microfuge tube and 0.2 ml extraction buffer (2% Triton X-100, 1% SDS, 100 mM NaCl, 10 mM Tris-HCl pH 8.0, 1 mM EDTA), 0.2 ml phenol-chloroform-isoamyl alcohol (25:24:1), and 0.3 g glass beads (0.3 mm in diameter) were added to the pellet. The mixture was vortexed at high speed for 2 min and then centrifuged at top speed for 5 min. The aqueous layer was transferred to a new tube and the nucleic acid was precipitated with 1 ml of isopropanol and washed twice with 70% ethanol. The resulting pellet was resuspended in 20 µl sterile water. Five µl of this DNA were used for transformation of E.coli.

### Plant material and growth conditions

Arabidopsis thaliana L. Heynh., ecotoype Col-0 was grown in axenic culture or in the greenhouse both with 14 h of light and 8 h of dark at 21.5°C. For axenic culture, seeds were surface sterilized for 5 min in 70% alcohol and 20 min in 1.2% sodium hypochlorite/0.01% Triton X-100, rinsed twice with sterile water and vernalized for 24 h at 4°C. Plants were grown on Murashige and Skoog (MS) medium (29) supplemented with 20 g/l sucrose and solidified with 7.5 g/l Bitek agar in parafilm-sealed petri dishes in a climate chamber.

### Plant Transformation

Plant transformations were carried out according to the floral dip protocol (30) with the following modifications: The infiltration medium contained 5% w/v sucrose, 0.03% (v/v) Silwet L-77 (OSi Specialities Inc., Danbury, CA, USA) and 44 nM benzylaminopurine. Plants were infiltrated by applying vacuum for approximately 5 min using a water pump.

### Selection of transformants

For hygromycin selection, plants were grown in axenic culture as described above on medium supplemented with 50 µg/ml hygromycin. After 2 weeks, transformants were identified by root growth and primary leaf development. These were transferred to soil and grown in 6 cm pots for seed prodution.

For Basta selection, seeds were vernalized for 24 hours at 4°C, sown in soil in 35 x 50 cm trays and transferred to the greenhouse. At the four leaf stage the trays were sprayed twice with BASTA ^{R} (0.1% in water, Aventis, Strasbourg, France). Resistant plants were selected and transferred to 6 cm pots.

### DNA extraction from plant material

DNA preparation was performed following the instructions of the Thomas Jack laboratory
(http://www.dartmouth.edu/⁻tjack/TAILDNAprep.html) except that an electrically driven steel pestle was used instead of a plastic mini pestle.

### DNA amplification by PCR

PCR reactions were carried out in a volume of 100 µl using 2 U Pfu Polymerase (Stratagene, LA Jolla, CA, USA) with Perkin-Elmer GeneAmp^{R}PCR Systems 2400 or 9700 (PE Applied Biosystems, Foster City, CA).

Annealing temperatures were set according to the primer-suppliers recommendations, other PCR conditions were optimized for maximum product yield. The typical PCR programm consisted of 25 cycles. DNA sequencing was performed by GATC GmbH, Konstanz, Germany.

### Experiment 1

### Isolation of the cDNA of the Arabidopsis thaliana P5CDH

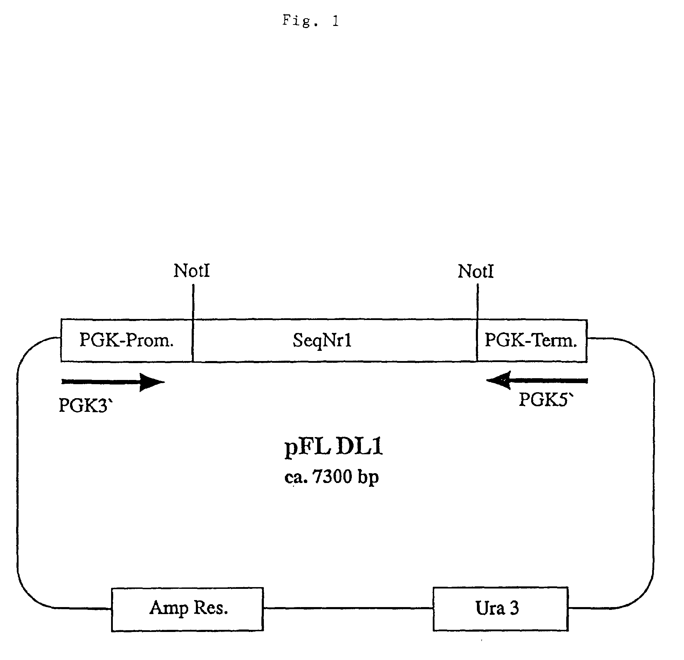

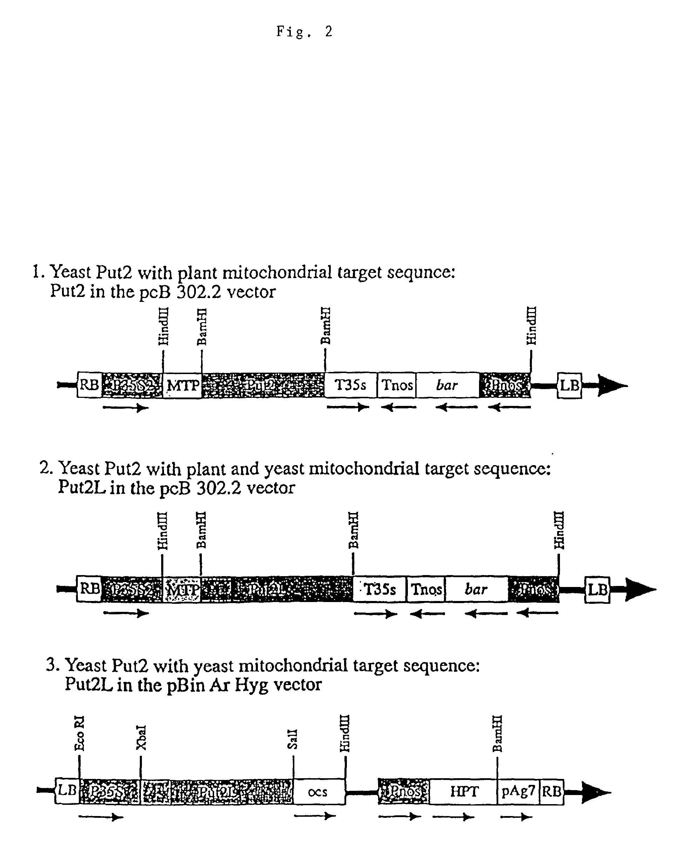

For the complementation of the yeast Put2 mutation in the yeast strain MB1472, a cDNA library of complete young Arabidopsis thaliana seedlings (two leave stage) in the yeast expression vector pFL61 (31) which had been made available by Minet (32) was used (Fig. 1).

The mutant yeast strain MB1472 was transformed with about 20 µg of the vector containing inserted Arabidopsis cDNAs using the lithium acetate/single-stranded carrier DNA/polyethylene glycol (LiAc/ss-DNA/PEG) transformation method (28). Yeast transformants which could now grow on synthetic Minimal medium (SD-Medium supplemented with 2 % glucose) with 0.1 % proline as sole nitrogen source were propagated and the plasmids were isolated according to standard methods. The resulting plasmids could complement the yeast mutation in Put2 after retransformation and were sequenced. The size of the corresponding cDNA is approximately 1.9kbp.
Sequencing of the cDNA inserted into the isolated library plasmid and comparison to sequence in the GenBank using the Blast webserver (NCBI, Marryland, USA) revealed that this cDNA derived from a transcript of the gene K19B1.9 predicted by the KAZUSA DNA Research Institute, Japan. The cDNA has strong similarity to the fisl mRNA from flax (Linum usitatissimum) with 73.3 % identical nucleotide residues (33). The deduced amino acid sequence is 79.9 % homologous to the deduced protein sequence of Fis1. Sequence Analysis of the primary structure of AtPut2 by the Profile Scan web server (ISREC, Switzerland) revealed a conserved motive for the cysteine active site of aldehyde dehydrogenases consisting of the amino acid residues 329-340. Comparison with other aldehyde dehydrogenases revealed that the central motive GQ(K/R)CSAx(S/R) is shared only by P5C dehydrogenases, and therein all known eukaryotic PSCDHs share the motive GQKCSAxS.

### Experiment 2

### Overexpressing of yeast Put2 in plants

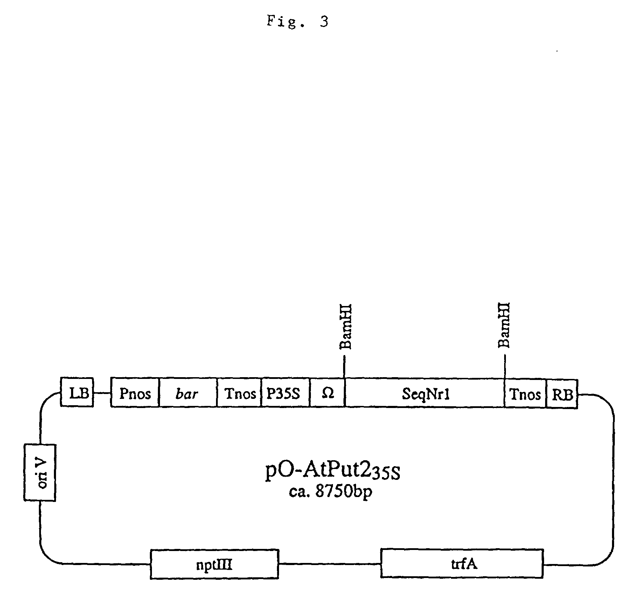

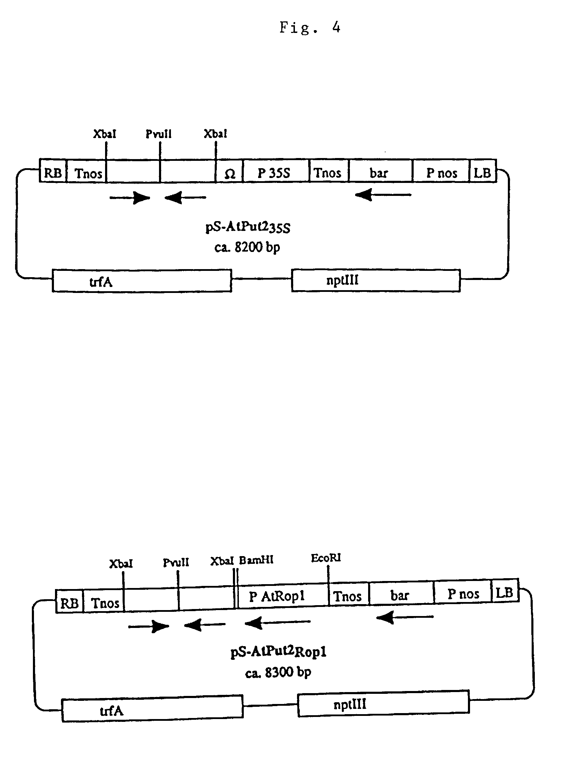

To overexpress the yeast Put2 gene (encoding the P5C dehydrogenase) in plants, gene specific primers were designed (Primers used: Put2rv: gcggatccttttTTATTCATAATTCGATGG; Put2fw:
gaggatccGAACCTGTTAAGCCATTTAG; Put2lfw: gaggatccattatgctatcagcaaggtg) and the coding region was amplified via PCR. Two PCR products of different size were amplified, one including the mitochondrial target sequence (Primers Put2-rv, Put2-lfw) and one excluding it (Primers Put2-rv, Put2-fw). The resulting fragments were cut with BamHI and cloned into the vector pCB302.2, leading to one construct with the plant and yeast mitochondrial target sequence and another one only with the plant mitochondrial target sequence (Fig. 2).
For a third construct containing only the yeast mitochondrial target sequence, another vector, pBinAr-Hyg, was used. The fragment without mitochondrial target sequence was ligated into the BamHI site of pBS KS⁻ (Stratagene) and was cut out again with SalI and XbaI and ligated into the vector pBinAr-Hyg previously cut with XbaI and SalI.
A. tumefaciens strain pGV2260 was transformed with these vectors and used for transformation of Arabidopsis plants using the floral dip method. Transformants were selected by resistance to Basta^{R} or hygromycin.

### LITERATURE

1. Wyn Jones, R.G., Storey, R., Leigh, R.A., Ahmad, N., and Pollard, A. 1977. A hypothesis on cytoplasmic osmoregulation. E Marr, O Ciferri, eds, Regulation of Cell Membrane Activities in Plants. Elsevier/North-Holland Biomedical Press, Amsterdam, 121-136
2. Schobert, B., and Tschesche, H. 1978. Unusual solution properties of proline and its interaction with proteins. Biochim Biophys Acta 541: 270-277
3. Smirnoff, N., and Cumbes, Q.J. 1989. Hydroxyl radical scavenging activity of compatible solutes. Phytochemistry 28: 1057-1060
4. Blum, A., and Ebercon, A. 1976. Genotypic responses in sorghum to drought stress. III. Free proline accumulation and drought resistance. Crop Sci 16: 428-431
5. Ahmad, I., and Hellebrust, J.A. 1988. The relationship between inorganic nitrogen metabolism and proline accumulation in osmoregulatory responses of two eurythaline microalgae. Plant Physiol 88: 348-354
6. Rhodes, D., Handa, S., and Bressan, R.A. 1986. Metabolic changes associated with adaptation of plant cells to water stress. Plant Physiol 82: 890-903
7 Voetberg, G.S., and Sharp, R.E. 1991. Growth at the maize primary root at low water potential. III. Role of increased proline deposition in osmotic adjustment. Plant Physiol 96: 1125-1130
8. Rentsch, D., Hirner, B., Schmelzer, E., and Frommer, W.B. 1996. Salt stress-induced proline transporters and salt stress-repressed broad specific amino acid permeases identified by suppression of a yeast amino acid targeting mutant. Plant Cell 8: 1437-1446
9. Schwacke, R., Grallath, S., Breitkreuz, K.E., Stransky, E., Stransky, H., Frommer, W.B., and Rentsch, D. 1999. LeProT1, a transporter for proline, glycine-betaine and gamma-aminobutyric acid in tomato pollen. Plant Cell 11: 377-391
10. Verbruggen, N., Villarroel, R., and Van Montagu, M. 1993. Osmoregulation of a pyrroline-5-carboxylate reductase gene in Arabidopsis thaliana. Plant Physiol 103: 771-781
11. Savoure, A., Jaoua, S., Hua, X.J., Ardiles, W., Van Montagou, M., and Verbruggen, N. 1995. Isolation, characterization, and chromosomal location of a gene encoding the DELTA-1-pyrroline-5-carboxylate synthetase in Arabidopsis thaliana. FEBS Lett 372: 13-19
12. Nanjo, T., Kabayashi, M., Yoshiba, Y., Sanada, Y., Wada, K., Tsukaya, H., Kakubari, Y., Yamaguchi-Shinozaki, K., and Shinozaki, K. 1999. Biological functions of proline in morphogenesis and osmotolerance revealed in antisense transgenic Arabidopsis thaliana. Plant J 18: 185-193
13. Hare, P.D., Cress, W.A., and van Staden, J. 1999. Proline synthesis and degradation: a model system for elucidating stress-related signal transduction. J Exp Bot 50: 413-434
14. Kiyosue, T., Yoshiba, Y., Yamaguchi-Shinozaki, K., and Shinozaki, K. 1996. A nuclear gene encoding mitohondrial proline dehydrogenase, an enzyme involved in proline metabolism, is upregulated by proline but downregulated by dehydration in A. thaliana. Plant Cell 8: 1323-1335
15. Larher, F., Leport, L., Petrivalsky, M., and Cappart M. 1993. Effectors for the osmoinduced proline response in higher plants. Plant Physiol Biochem 31: 911-922
16. Pesci, P. 1993. Glucose mimics the enhancing effect of light on ABA-induced proline accumulation in hydrated barley and wheat leaves. J Plant Physiol 142: 355-359
17. Balibrea, M.E., Rus-Alvarez, A.M., Bolarin, M.C., and Perez-Alfocea, F. 1997. Fast changes in soluble carbohydrates and proline contents in tomato seedlings in response to ionic and non-ionic iso-osmotic stresses. J Plant Physiol 151: 221-226
18. Chen, D.M., Keiper, F.J., and De Filippis, L.F. 1998. Physiological changes accompanying the induction of salt tolerance in Eucalyptus microcorys shoots in tissue culture. J Plant Physiol 152: 555-563
19. Clifford, S.C., Arndt, S.K., Corlett, J.E., Joshi, S., Sankhla, N., Popp, M., and Jones, H.G. 1998. The role of solute accumulation, osmotic adjustment and changes in cell wall elasticity in drought tolerance in Ziziphus mauritiana (Lamk.). J Exp Bot 49: 967-977
20. Hellmann, H., Funck, D., Rentsch, D., and Frommer, W.B. 2000. Plant Physiol 122: 357-367
21. Peng, Z., Lu, Q., and Verma, D.P.S. 1996. Reciprocal regulation of DELTA-1-pyrroline-5-carboxylate synthetase and proline dehydrogenase genes controls proline levels during and after osmotic stress in plants. Mol Gen Genet 253: 334-341
22. Ausubel, F.M., Brent, R., Kingston, R.E., Moore, D.D., Seidman, J.G., Smith, J.A., and Struhl, K. 1987. Current Protocols in Molecular Biology, Wiley Interscience
23. Donald, S.P., Sun, X.Y., Mei, J.M., and Phang, J.M. 1999. p53-dependent induction of proline oxidase and generation of reactive oxygen species in adriamycin-induced apoptosis. Proceedings American Association for Cancer Research Annual Meeting. 646
24. Maniatis, T., Fritsch, E.F., and Sambrook, J. 1996. Molecular Cloning: A Laboratory Manual, Cold Spring Harbor, NY: Cold Spring Harbor Laborator
25. Deblaire, R., Bytebier, B., Greve, H., Deboeck, F., Schell, J., Van Montagu, M., and Leemans, J. 1985. Efficient octopine Ti plasmid-derived vectors for Agrobacterium-mediated gene transfer to plants. Nucleic Acids Res 13: 4777-4788
26. Vervliet,G., Holsters, M., Teuchy, H., Van Montagu, M., and Schell, J. 1975. Characterization of different plaque-forming and defective temperate phages in Agrobacterium. J Gen Virol 26: 33-48
27. Güldener, U., Heck, S., Fielder, T., Beinhauer, J., and Hegemann, J.H. 1996. A new efficient gene disruption cassette for repeated use in budding yeast. Nucleic Acids Res 24: 2519-2524
28. Agatep, R., Kirkpatrick, R.D., Parchaliuk, D.L., Woods, R.A., and Gietz, R.D. 1999. Transformation of Saccharomyces cerevisiae by the lithium acetate/sigle-stranded carrier DNA/polyethylene glycol /LiAc/ss-DNA/PEG protocol. Technical Tips Online: http://www.tto.trends.com
29. Murashige, T., and Skoog, F. 1962. A revised medium for rapid growth and bioassays with tobacco tissue cultures. Physiol Plant 15: 473-497
30. Clough, S.J., and Bent, A.F. 1998. Floral dip: a simplified method for Agrobacterium-mediated transformation of Arabidopsis thaliana. Plant J 16: 735-743
31. Minet, M., and Lacroute, F. 1990. Cloning and sequencing of a human cDNA coding for a multifunctional polypeptide of the purine pathway by complementation of the ade2-101 mutant in Saccharomyces cerevisiae. Curr Genet 18: 287-291
32. Minet, M., Dufour, M.E., and Lacroute, F. 1992. Complementation of Saccharomyces cerevisiae auxotrophic mutants by Arabidopsis thaliana cDNAs. Plant J 2:417-422
33. Roberts, J.K., and Pryor, A. 1995. Isolation of a flax (Linum usitatissimum) gene induced during susceptible infection by flax rust (Melampsora lini). Plant J 8: 1-8

## Claims

1. Nucleic acid molecule encoding an amino acid sequence naturally occuring in plants with a pyrroline-5-carboxylate dehydrogenase activity.

2. Nucleic acid molecule, particularly according to claim 1, **characterized in that** it comprises at least a part of the sequence according to SEQ ID NO. 1 or the complementary strand thereof.

3. Nucleic acid molecule, particularly according to claim 1 or claim 2, **characterized in that** it is at least 60%, particularly at least 80% and more particularly at least 90%, identical to at least a part of the sequence according to SEQ ID NO. 1 or the complementary strand thereof.

4. Nucleic acid molecule, particularly according to one of the preceding claims, **characterized in that** it hybridizes with at least a part of the sequence according to SEQ ID NO. 1 or the complementary strand thereof.

5. Nucleic acid molecule according to one of the preceding claims, **characterized in that** it encodes at least a part of an amino acid sequence according to SEQ ID NO. 2.

6. Nucleic acid molecule according to one of the preceding claims, **characterized in that** it is a DNA molecule, especially a cDNA or genomic DNA molecule, or a RNA molecule.

7. Nucleic acid molecule according to one of the preceding claims, **characterized in that** it is combined with a regulatory element, especially a promotor, preferably a promotor suited for organ specific expression in organisms, especially plants.

8. Vector, comprising a nucleic acid molecule according to one of the preceding claims or fragments thereof.

9. Host cell, comprising at least one nucleic acid molecule according to one of claims 1 to 7 and/or at least one vector according to claim 8.

10. Genetically engineered cell, especially plant cell, transformed by at least one nucleic acid molecule according to one of claims 1 to 7 and/or by at least one vector according to claim 8, or a cell derived from said cell.

11. Amino acid sequence comprising at least a part of the sequence according to SEQ ID NO. 2, or a derivative thereof.

12. Method for screening plants or parts thereof, especially plant cells, with respect to pyrroline-5-carboxylate dehydrogenase activity, comprising the following steps:
a) cultivating plants or parts thereof on proline- and/or pyrroline-5-carboxylate-containing substrate,
b) monitoring sensitivity against proline or pyrroline-5-carboxylate and as a consequence P5CDH activity.

13. Method for the development of an active substance, especially a fungicide and/or herbicide, comprising the following steps:
a) transforming cells, especially yeast cells deficient in endogenous pyrroline-5-carboxylate dehydrogenase activity, with a nucleic acid molecule encoding an amino acid sequence naturally occuring in plants with a pyrroline-5-carboxylate dehydrogenase activity,
b) incubating the cells with a substance potentially altering the activity of the pyrroline-5-carboxylate dehydrogenase transformed into the cells, and
c) monitoring the activity of the pyrroline-5-carboxylate dehydrogenase in order to identify active substance.

14. Method according to claim 13, **characterized in that** the monitoring according to step c) is performed by monitoring the growth on proline- and/or pyrroline 5 carboxylate-containing substrate.

15. Use of at least one nucleic acid molecule according to one of claims 1 to 7, at least one vector according to claim 8 and/or at least one amino acid sequence according to claim 11 as target for the development of an active substance, especially a fungicide and/or herbicide, particularly by performing a method according to claim 13 or 14.

16. Use of at least one nucleic acid molecule according to one of claims 1 to 7 or at least one vector according to claim 8 or at least one amino acid sequence according to claim 11 for investigation of proline pathways, especially degradation of proline, in organisms, especially plants.

17. Use according to claim 16, **characterized in that** direct or indirect modulators, especially inhibitors, of pyrroline-5-carboxylate dehydrogenase are to be identified.


SEQUENCE LISTING

<110> SYMPORE GmbH

<120> A NEW PLANT PROTEIN

<130> A 35204 EP

<140> EP 00 11 6936.6
<141> 2000-08-04

<160> 2

<170> PatentIn Ver. 2.1

<210> 1
<211> 2087
<212> DNA
<213> Arabidopsis thaliana

<220>
<221> CDS
<222> (126)..(1796)
<223> ORF of AtPut2 in pFL61

<220>
<221> transit_peptide
<222> (126)..(165)
<223> Mitochondrial target sequence

<400> 1
aaatataaaa ccagcggccg ccagtgtgag taatttagat tcgctatttt attaccaatc 60

ttcttgaatc ctctcatcca atcgtctgat tctcatcttt ctccgttact tgtggatctt 120

acgca atg tat aga gtt ttt gcg agc cga gca ctg aga gct aag tct ctc 170
      Met Tyr Arg Val Phe Ala Ser Arg Ala Leu Arg Ala Lys Ser Leu
        1               5                  10                  15

tgt gac aaa tca tcc acc tcg ctt gct tct ttg act ctc tcc aga ttg 218
Cys Asp Lys Ser Ser Thr Ser Leu Ala Ser Leu Thr Leu Ser Arg Leu
                 20                  25                  30

aat cat tcc ata cct ttt gct acc gta gat gct gag gag cta tct ggt 266
Asn His Ser Ile Pro Phe Ala Thr Val Asp Ala Glu Glu Leu Ser Gly
             35                  40                  45

tct cac cct gct gaa gta cag agc ttt gtg cag gga aag tgg ata gga 314

Ser His Pro Ala Glu Val Gln Ser Phe Val Gln Gly Lys Trp Ile Gly
        50                  55                  60

tcg tca aat cat aac acg ctt cta gat cct cta aat gga gaa cct ttt     362
Ser Ser Asn His Asn Thr Leu Leu Asp Pro Leu Asn Gly Glu Pro Phe
    65                  70                  75

atc aaa gtt gct gaa gtg gat gaa tca gga act cag cca ttt gtg gac     410
Ile Lys Val Ala Glu Val Asp Glu Ser Gly Thr Gln Pro Phe Val Asp
80                  85                  90                  95

agc tta tcc cag tgc ccg aaa cat ggt ctc cat aac cct ttc aaa tct     458
Ser Leu Ser Gln Cys Pro Lys His Gly Leu His Asn Pro Phe Lys Ser
                100                 105                 110

cca gag aga tac ctt tta tat gga gat atc tcg aca aag gca gct cat     506
Pro Glu Arg Tyr Leu Leu Tyr Gly Asp Ile Ser Thr Lys Ala Ala His
            115                 120                 125

atg ctc gcc tta cct aag gtt gct gat ttc ttc gcg agg ttg att caa     554
Met Leu Ala Leu Pro Lys Val Ala Asp Phe Phe Ala Arg Leu Ile Gln
        130                 135                 140

agg gtt gct cca aag agt tac cag caa gct gct gga gaa gtc ttt gtc     602
Arg Val Ala Pro Lys Ser Tyr Gln Gln Ala Ala Gly Glu Val Phe Val
    145                 150                 155

aca agg aag ttt tta gaa aac ttt tgt ggt gat cag gtg cgg ttc cta     650
Thr Arg Lys Phe Leu Glu Asn Phe Cys Gly Asp Gln Val Arg Phe Leu
160                 165                 170                 175

gca cgg tct ttt gca atc cct ggg aat cat ctt ggg cag caa agt cat     698
Ala Arg Ser Phe Ala Ile Pro Gly Asn His Leu Gly Gln Gln Ser His
                180                 185                 190

ggt tac cgt tgg cct tat ggt cct gta acg att gtt aca cca ttt aat     746
Gly Tyr Arg Trp Pro Tyr Gly Pro Val Thr Ile Val Thr Pro Phe Asn
            195                 200                 205

ttt cca ctt gag att cca cta ctt cag ttg atg ggg gca tta tac atg     794
Phe Pro Leu Glu Ile Pro Leu Leu Gln Leu Met Gly Ala Leu Tyr Met
        210                 215                 220

ggt aac aaa cct ctt ctt aaa gtg gac agc aag gta agc att gta atg     842
Gly Asn Lys Pro Leu Leu Lys Val Asp Ser Lys Val Ser Ile Val Met
    225                 230                 235

gag caa atg atg cga ttg ctc cac tat tgt ggt tta ccc gct gaa gat     890

Glu Gln Met Met Arg Leu Leu His Tyr Cys Gly Leu Pro Ala Glu Asp
240                 245                 250                 255

gtc gac ttt ata aat tct gat ggg aag aca atg aac aag ata ttg tta     938
Val Asp Phe Ile Asn Ser Asp Gly Lys Thr Met Asn Lys Ile Leu Leu
                260                 265                 270

gag gcg aat cca cgg atg acc ctc ttc acg ggt agt tct aga gta gct     986
Glu Ala Asn Pro Arg Met Thr Leu Phe Thr Gly Ser Ser Arg Val Ala
            275                 280                 285

gaa aag ttg gca ctt gac ctt aag ggt cga atc aga tta gaa gat gct     1034
Glu Lys Leu Ala Leu Asp Leu Lys Gly Arg Ile Arg Leu Glu Asp Ala
        290                 295                 300

gga ttt gat tgg aaa gtt tta gga cca gat gtt cag gag gtt gat tac     1082
Gly Phe Asp Trp Lys Val Leu Gly Pro Asp Val Gln Glu Val Asp Tyr
    305                 310                 315

gtt gca tgg caa tgt gat caa gat gca tat gcg tgc agt gga cag aag     1130
Val Ala Trp Gln Cys Asp Gln Asp Ala Tyr Ala Cys Ser Gly Gln Lys
320                 325                 330                 335

tgt tct gca cag tct atg ctt ttt gtt cat gag aac tgg tca aaa acc     1178
Cys Ser Ala Gln Ser Met Leu Phe Val His Glu Asn Trp Ser Lys Thr
                340                 345                 350

cct ctt gtt tcc aaa tta aaa gaa cta gcg gaa aga cgc aaa ctg gaa     1226
Pro Leu Val Ser Lys Leu Lys Glu Leu Ala Glu Arg Arg Lys Leu Glu
            355                 360                 365

gac tta acc att ggt cct gtc ctt aca ttt aca act gag gca atg ttg     1274
Asp Leu Thr Ile Gly Pro Val Leu Thr Phe Thr Thr Glu Ala Met Leu
        370                 375                 380

gag cac atg gag aat ctg ctt cag att cct ggc tca aag cta ctc ttt     1322
Glu His Met Glu Asn Leu Leu Gln Ile Pro Gly Ser Lys Leu Leu Phe
    385                 390                 395

ggt ggc aag gaa ttg aag aat cat tcg att cct tca atc tac gga gct     1370
Gly Gly Lys Glu Leu Lys Asn His Ser Ile Pro Ser Ile Tyr Gly Ala
400                 405                 410                 415

ttg gag cca act gca gtt tat gtt ccc att gaa gaa ttc ttg aag gat     1418
Leu Glu Pro Thr Ala Val Tyr Val Pro Ile Glu Glu Phe Leu Lys Asp
                420                 425                 430

aat aaa acc tac gaa ctc gtc acc aaa gaa atc ttt gga cca ttt cag     1466

Asn Lys Thr Tyr Glu Leu Val Thr Lys Glu Ile Phe Gly Pro Phe Gln
            435                 440                 445

att gta acg gaa tac aaa aag gat caa ctt cct cta gtg ttg gac gca    1514
Ile Val Thr Glu Tyr Lys Lys Asp Gln Leu Pro Leu Val Leu Asp Ala
        450                 455                 460

ttg gag agg atg cac gct cat cta act gca gct gtt gtt tca aac gat    1562
Leu Glu Arg Met His Ala His Leu Thr Ala Ala Val Val Ser Asn Asp
    465                 470                 475

ccc atc ttt ctt cag gag gta ata ggg aac tca gtg aat ggg act aca    1610
Pro Ile Phe Leu Gln Glu Val Ile Gly Asn Ser Val Asn Gly Thr Thr
480                 485                 490                 495

tat gct gga cta aga gga aga aca act gga gct cct cag aat cac tgg    1658
Tyr Ala Gly Leu Arg Gly Arg Thr Thr Gly Ala Pro Gln Asn His Trp
                500                 505                 510

ttt gga cct gcg ggg gac ccg aga ggg gcc ggg ata ggg aca cca gag    1706
Phe Gly Pro Ala Gly Asp Pro Arg Gly Ala Gly Ile Gly Thr Pro Glu
            515                 520                 525

gct ata aag ctg gtt tgg tca tgc cac aga gaa gtc atc tac gat tat    1754
Ala Ile Lys Leu Val Trp Ser Cys His Arg Glu Val Ile Tyr Asp Tyr
        530                 535                 540

ggt ccg gtt cca caa ggt tgg gaa ctt cct cca tct act tag            1796
Gly Pro Val Pro Gln Gly Trp Glu Leu Pro Pro Ser Thr
    545                 550                 555

aagtggtgtg tccactaata attaataaac taagatctta gtgtgctgtt gcatcaaata 1856

aatatgagtt ttgtagtttt atctctacga gtagtttccg tgtgagggaa taagagcttg 1916

aaacatgaag ttttcaacga cgttgactta gtcaaggggt agagggatta agacagagag 1976

agaactattg taaaactcag tccaccagaa ttttcttcag ctcaaagcca gacaataata 2036

caaatgtttt tgaaaaaaaa aaaaaaacta aattactcac actggcgccg c          2087

<210> 2
<211> 556
<212> PRT
<213> Arabidopsis thaliana

<400> 2

Met Tyr Arg Val Phe Ala Ser Arg Ala Leu Arg Ala Lys Ser Leu Cys
1               5                   10                  15
Asp Lys Ser Ser Thr Ser Leu Ala Ser Leu Thr Leu Ser Arg Leu Asn
            20                  25                  30
His Ser Ile Pro Phe Ala Thr Val Asp Ala Glu Glu Leu Ser Gly Ser
        35                  40                  45
His Pro Ala Glu Val Gln Ser Phe Val Gln Gly Lys Trp Ile Gly Ser
    50                  55                  60
Ser Asn His Asn Thr Leu Leu Asp Pro Leu Asn Gly Glu Pro Phe Ile
65                  70                  75                  80
Lys Val Ala Glu Val Asp Glu Ser Gly Thr Gln Pro Phe Val Asp Ser
                85                  90                  95
Leu Ser Gln Cys Pro Lys His Gly Leu His Asn Pro Phe Lys Ser Pro
            100                 105                 110
Glu Arg Tyr Leu Leu Tyr Gly Asp Ile Ser Thr Lys Ala Ala His Met
        115                 120                 125
Leu Ala Leu Pro Lys Val Ala Asp Phe Phe Ala Arg Leu Ile Gln Arg
    130                 135                 140
Val Ala Pro Lys Ser Tyr Gln Gln Ala Ala Gly Glu Val Phe Val Thr
145                 150                 155                 160
Arg Lys Phe Leu Glu Asn Phe Cys Gly Asp Gln Val Arg Phe Leu Ala
                165                 170                 175
Arg Ser Phe Ala Ile Pro Gly Asn His Leu Gly Gln Gln Ser His Gly
            180                 185                 190
Tyr Arg Trp Pro Tyr Gly Pro Val Thr Ile Val Thr Pro Phe Asn Phe
        195                 200                 205
Pro Leu Glu Ile Pro Leu Leu Gln Leu Met Gly Ala Leu Tyr Met Gly
    210                 215                 220
Asn Lys Pro Leu Leu Lys Val Asp Ser Lys Val Ser Ile Val Met Glu
225                 230                 235                 240
Gln Met Met Arg Leu Leu His Tyr Cys Gly Leu Pro Ala Glu Asp Val
                245                 250                 255
Asp Phe Ile Asn Ser Asp Gly Lys Thr Met Asn Lys Ile Leu Leu Glu
            260                 265                 270
Ala Asn Pro Arg Met Thr Leu Phe Thr Gly Ser Ser Arg Val Ala Glu
        275                 280                 285
Lys Leu Ala Leu Asp Leu Lys Gly Arg Ile Arg Leu Glu Asp Ala Gly
    290                 295                 300
Phe Asp Trp Lys Val Leu Gly Pro Asp Val Gln Glu Val Asp Tyr Val
305                 310                 315                 320
Ala Trp Gln Cys Asp Gln Asp Ala Tyr Ala Cys Ser Gly Gln Lys Cys
                325                 330                 335
Ser Ala Gln Ser Met Leu Phe Val His Glu Asn Trp Ser Lys Thr Pro
            340                 345                 350
Leu Val Ser Lys Leu Lys Glu Leu Ala Glu Arg Arg Lys Leu Glu Asp
        355                 360                 365
Leu Thr Ile Gly Pro Val Leu Thr Phe Thr Thr Glu Ala Met Leu Glu
    370                 375                 380

His Met Glu Asn Leu Leu Gln Ile Pro Gly Ser Lys Leu Leu Phe Gly
385                 390                 395                 400
Gly Lys Glu Leu Lys Asn His Ser Ile Pro Ser Ile Tyr Gly Ala Leu
                405                 410                 415
Glu Pro Thr Ala Val Tyr Val Pro Ile Glu Glu Phe Leu Lys Asp Asn
            420                 425                 430
Lys Thr Tyr Glu Leu Val Thr Lys Glu Ile Phe Gly Pro Phe Gln Ile
        435                 440                 445
Val Thr Glu Tyr Lys Lys Asp Gln Leu Pro Leu Val Leu Asp Ala Leu
    450                 455                 460
Glu Arg Met His Ala His Leu Thr Ala Ala Val Val Ser Asn Asp Pro
465                 470                 475                 480
Ile Phe Leu Gln Glu Val Ile Gly Asn Ser Val Asn Gly Thr Thr Tyr
                485                 490                 495
Ala Gly Leu Arg Gly Arg Thr Thr Gly Ala Pro Gln Asn His Trp Phe
            500                 505                 510
Gly Pro Ala Gly Asp Pro Arg Gly Ala Gly Ile Gly Thr Pro Glu Ala
        515                 520                 525
Ile Lys Leu Val Trp Ser Cys His Arg Glu Val Ile Tyr Asp Tyr Gly
    530                 535                 540
Pro Val Pro Gln Gly Trp Glu Leu Pro Pro Ser Thr
545                 550                 555